Europäisches Patentamt

European Patent Office

Office européen des brevets

(19)

(11) **EP 0 819 420 B1**

(12) **FASCICULE DE BREVET EUROPEEN**

(45) Date de publication et mention
de la délivrance du brevet:
**05.01.2000 Bulletin 2000/01**

(51) Int. Cl.[7]: **A61K 7/043**

(21) Numéro de dépôt: **97401419.3**

(22) Date de dépôt: **19.06.1997**

(54) **Utilisation de l'acide silicique colloidal dans une composition de vernis-à-ongles**

Verwendung von kolloidaler Kieselsäure in einer Nagellackzusammensetzung

Use of colloidal silicic acid in a fingernail composition

(84) Etats contractants désignés:
**DE ES FR GB IT**

(30) Priorité: **18.07.1996 FR 9609010**

(43) Date de publication de la demande:
**21.01.1998 Bulletin 1998/04**

(73) Titulaire: **L'OREAL**
**75008 Paris (FR)**

(72) Inventeurs:
• **Ramin, Roland**
**91760 Itteville (FR)**
• **Garson, Jean-Claude**
**92150 Suresnes (FR)**

(74) Mandataire:
**Dodin, Catherine et al**
**L'Oreal-D.P.I.,**
**6, rue Bertrand Sincholle**
**92585 Clichy Cédex (FR)**

(56) Documents cités:
**EP-A- 0 063 467      EP-A- 0 453 628**
**EP-A- 0 504 754      EP-A- 0 745 372**
**FR-A- 2 578 741      GB-A- 884 626**
**US-A- 5 071 639**

• **CHEMICAL ABSTRACTS, vol. 123, no. 10, 4 septembre 1995 Columbus, Ohio, US; abstract no. 122756, XP002028841 & JP 07 138 131 A (YUHO CHEMICALS INC.) 30 mai 1995**
• **PATENT ABSTRACTS OF JAPAN vol. 96, no. 8, 9 avril 1996 & JP 08 092038 A (KAO CORP.)**
• **CHEMICAL ABSTRACTS, vol. 121, no. 26, 26 décembre 1994 Columbus, Ohio, US; abstract no. 308008, XP002028846 & JP 06 227 945 A (YUHO CHEMICALS INC.) 16 août 1994**

**Description**

La présente invention a trait à l'utilisation d'acide silicique colloïdal dans une composition de vernis à ongles en milieu solvant organique, aqueux ou huileux.

On connaît des compositions à appliquer par exemple sur l'ongle, de type vernis à ongles ou base de soin pour ongles en milieu solvant, comprenant de manière usuelle, au moins un polymère filmogène, éventuellement un agent plastifiant, des pigments, des agents rhéologiques et des solvants.

Une telle composition peut ainsi permettre d'embellir l'ongle. Toutefois, les compositions connues jusqu'à présent présentent le défaut de s'user rapidement, ce qui contraint l'utilisatrice à renouveler à des intervalles de temps très rapprochés, l'application d'une nouvelle couche de vernis après élimination de la couche abîmée.

Ce défaut des bases de soin pour ongles et des vernis à ongles a conduit la demanderesse à rechercher des agents qui, incorporés dans une composition de vernis ou de base de soin pour les ongles, conféreraient aux films obtenus à partir de celle-ci une plus grande dureté superficielle et, par conséquent, augmenteraient sa résistance aux frottements et/ou aux chocs.

Il est connu du document US-A-5071639 une composition de vernis-à-ongles comprenant une montmorillonite organomodifiée, de la silice, un tensioactif anionique et un solvant non aromatique. Le document JP-A-8-092038 décrit un vernis-à-ongles comprenant une émulsion de polymère synthétisée en présence de silice colloïdale.

La demanderesse a constaté que, de manière surprenante, l'acide silicique colloïdal pouvait être utilisé de manière satisfaisante pour améliorer la dureté superficielle des films obtenus à l'aide des vernis et bases de soin pour l'ongle usuels.

Ainsi, la présente invention a pour objet l'utilisation de l'acide silicique colloïdal en tant qu'agent durcissant dans une composition de vernis ou de base de soin pour ongles comprenant au moins une matière filmogène et au moins un solvant organique et/ou aqueux et/ou huileux.

On entend dans la présente description par 'agent durcissant', un agent susceptible d'améliorer la dureté superficielle d'un film d'une composition de vernis ou de base de soin déposé sur un support, notamment kératinique, tel qu'un ongle.

L'utilisation d'une composition selon l'invention sur les ongles permet donc l'obtention d'un film de vernis ou de base qui présente une meilleure dureté superficielle et par conséquent, une plus grande durée de vie (meilleure résistance aux agressions telles que les chocs, les frottements, les rayures, les pressions).

La composition pour l'utilisation selon l'invention comprend donc au moins une matière filmogène. Lorsque la base de soin ou le vernis selon l'invention est une composition en milieu solvant organique la matière filmogène peut être choisie, notamment, parmi les résines alkydes, acryliques et/ou vinyliques, les polyuréthannes et les polyesters, les celluloses et dérivés cellulosiques telles que la nitrocellulose, et les résines résultant de la condensation de formaldéhyde avec une arylsulfonamide, et leurs mélanges.

Dans ce cas, la matière filmogène est généralement en solution, par exemple à 5-25% en poids, dans un solvant organique tel qu'un hydrocarbure aromatique comme par exemple le toluène ou le xylène, un hydrocarbure aliphatique comme par exemple le n-heptane, un ester comme par exemple l'acétate d'éthyle, l'acétate de butyle, une cétone, comme par exemple l'acétone, la méthyl éthyl cétone, un alcool, comme par exemple l'éthanol, l'isopropanol et le butanol, ainsi que leurs mélanges.

Lorsque la base de soin ou le vernis à ongles pour l'utilisation selon l'invention est une composition en milieu aqueux, la composition peut comprendre le polymère sous forme de particules de polymère filmogène en dispersion. Parmi les polymères filmogènes susceptibles d'être utilisés, on peut citer les polyuréthannes, par exemple anioniques, les polyesters-polyuréthannes, les polyéther-polyuréthannes, les polymères radicalaires notamment de type acrylique, acrylique styrène et/ou vinylique, les polyesters, les résines alkydes, seuls ou en mélange.

La dispersion peut également comprendre un polymère associatif de type polyuréthanne ou une gomme naturelle telle que la gomme xanthane. La composition présente, de préférence, un taux de matière sèche de 25-45% en poids.

La composition peut également comprendre un agent plastifiant et éventuellement des agents rhéologiques. Parmi les agents plastifiants, on peut citer les citrates, les phtalates, les esters et/ou le camphre, généralement utilisés en une quantité de 5-30% en poids par rapport au poids de la composition.

Parmi les agents rhéologiques, on peut citer les bentonites organophiles, les dérivés de cellulose, les dérivés d'acide polyacrylique réticulé, les gommes de guar ou de caroube, ainsi que les gommes de xanthane.

L'acide silicique colloïdal susceptible d'être utilisé dans la composition pour l'utilisation selon l'invention est une silice pyrogénée ou traitée en surface, qui peut se présenter sous forme de silice pyrogénée hydrophile, de silice pyrogénée hydrophobe, ou de silice traitée en surface par un traitement organique.

Les silices pyrogénées peuvent être obtenues par hydrolyse à haute température d'un composé volatil du silicium dans une flamme oxhydrique. Il s'ensuit la production d'une silice finement divisée. Par réaction, on peut modifier chimiquement la surface de ladite silice, par réduction du nombre de groupes silanols afin d'obtenir une silice hydrophobe.

L'acide silicique colloïdal présente de préférence une taille des particules pouvant être nanométrique à micrométrique, par exemple de l'ordre de 10-200 nm. L'acide silicique colloïdal selon l'invention peut être entre autre choisi parmi les composés vendus par DEGUSSA, sous les marques Aerosil MOX80® ou Aerosil COK84® qui sont des silices spéciales, Aerosil R972® qui est une silice hydrophobe, ou encore Aerosil OK412® qui est une silice traitée en surface ou sous la marque Aerosil 200® qui est une silice hydrophile.

La composition pour l'utilisation selon l'invention peut comprendre l'acide silicique colloïdal en une quantité de 0,05 % à 5% en poids, de préférence en une quantité de 0,5 - 2 % en poids par rapport au poids total de la composition.

La composition pour l'utilisation selon l'invention peut en outre comprendre tout additif connu de l'homme du métier comme étant susceptible d'être incorporé dans une telle composition, tels que des agents d'étalement, des agents mouillants, des agents dispersants, des anti-mousses, des conservateurs, des filtres UV, des colorants, des pigments, des nacres, des actifs tels que le N-butylformal, le D-panthénol, le phytantriol, les vitamines et leurs dérivés, la kératine et ses dérivés, la mélanine, le collagène, la cystine, le chitosane et ses dérivés, les céramides, la biotine, les oligo-éléments, la glycérine, les hydrolysats de protéines, les phospholipides, les agents hydratants. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition pour l'utilisation selon l'invention peut être préparée par l'homme du métier sur la base de ses connaissances générales et selon l'état de la technique.

La composition pour l'utilisation selon l'invention peut se présenter sous la forme d'un produit à appliquer sur les ongles, tel qu'un vernis, une base ou une base de soin.

Les essais de dureté Vickers montrent une plus grande dureté des vernis selon l'invention par rapport aux vernis qui ne comportent pas de silice ou par rapport aux vernis qui comprennent d'autres additifs. L'invention est illustrée plus en détail dans les exemples suivants.

## EXEMPLES

On a déterminé la dureté d'un vernis comprenant de l'acide silicique colloïdal.

Principe:

On applique sur l'ongle une couche du vernis à tester et on attend la fin du séchage.

On applique sur l'ongle un pénétromètre en forme de pyramide à base carrée, à l'aide d'une charge P. On détermine ensuite la dimension moyenne d'une diagonale de l'empreinte carrée obtenue avec le pénétromètre. La dureté VICKERS (HV) est alors déterminée par la relation :

$$HV = \frac{1854,4 \times P}{d^2}$$

d = diagonale moyenne en μm
P = charge appliquée en g

La mesure de la dureté VICKERS est effectuée suivant la norme ASTM E384-89 à l'aide du microduromètre M 400 g 2 de la société LECO.

## Exemple 1:

On prépare un vernis à ongles ayant la composition suivante (% en poids) :

| | |
|---|---|
| . nitrocellulose | 15 % |
| . acétyl tributyl citrate | 6 % |
| . résine tosylamide/formaldéhyde | 10 % |
| . acide silicique colloïdal (AEROSIL 200 de DEGUSSA) | 1 % |
| . solvant (acétates d'éthyle et de butyle) qsp | 100 % |

On obtient un vernis de texture adéquate, qui s'applique aisément sur l'ongle. Il permet, après séchage, l'obtention d'un film lisse et homogène. Dureté Vickers : HV=5,8

## Exemple 2 : Vernis-témoin

On prépare un vernis à ongles ayant la composition suivante (% en poids) :

| | |
|---|---|
| . nitrocellulose | 15 % |
| . acétyl tributyl citrate | 6 % |
| . résine tosylamide/formaldéhyde | 10 % |
| . solvant (acétates d'éthyle et de butyle) | qsp 100 % |

Dureté Vickers : HV=4,8 **Exemple 3 : Vernis-témoin**
On prépare un vernis à ongles ayant la composition suivante (% en poids) :

| | |
|---|---|
| . nitrocellulose | 15 % |
| . acétyl tributyl citrate | 6 % |
| . résine tosylamide/formaldéhyde | 10 % |
| . hectorite stéaralkonium (nom CTFA) | 1 % |
| . solvant (acétates d'éthyle et de butyle) | qsp 100 % |

Dureté Vickers : HV=4,5
On constate que les vernis utilisant l'acide silicique colloïdal selon l'invention (exemple 1) donnent un film dont la dureté superficielle est supérieure à la dureté superficielle d'un film obtenu à partir d'une composition ne comprenant pas d'agent rhéologique (exemple 2) ou d'une composition comprenant un agent rhéologique du type bentone (exemple 3).

## Revendications

1. Utilisation de l'acide silicique colloïdal en tant qu'agent durcissant dans une composition de vernis ou de base de soin pour ongles comprenant au moins une matière filmogène et au moins un solvant organique et/ou aqueux et/ou huileux.

2. Utilisation de l'acide silicique colloïdal en tant qu'agent durcissant dans une composition de vernis ou de base de soin pour ongles comprenant au moins une matière filmogène et au moins un solvant organique et/ou aqueux et/ou huileux pour augmenter la durée de vie du film de vernis ou de base.

3. Utilisation de l'acide silicique colloïdal en tant qu'agent durcissant dans une composition de vernis ou de base de soin pour ongles comprenant au moins une matière filmogène et au moins un solvant organique et/ou aqueux et/ou huileux, pour améliorer la tenue d'un vernis ou d'une base, et/ou sa résistance aux frottements, à l'usure et/ou aux chocs.

4. Utilisation selon l'une des revendications précédentes, dans laquelle l'acide silicique colloïdal est choisi parmi les silices traitées en surface, les silices pyrogénées hydrophiles et/ou les silices pyrogénées hydrophobes.

5. Utilisation selon l'une des revendications précédentes, dans laquelle l'acide silicique colloïdal est présent en une quantité de 0,05 % à 5 % en poids, de préférence 0,5 - 2 % en poids, par rapport au poids total de la composition.

**6.** Utilisation selon l'une des revendications précédentes, caractérisée en ce que la composition de base de soin ou de vernis est une composition en milieu solvant organique.

**7.** Utilisation selon la revendication 6, caractérisée en ce que la matière filmogène est choisie parmi les résines alkydes, acryliques et/ou vinyliques, les polyuréthannes et les polyesters, les celluloses et dérivés cellulosiques telles que la nitrocellulose, et les résines résultant de la condensation de formaldéhyde avec une arylsulfonamide, et leurs mélanges.

**8.** Utilisation selon l'une des revendications 6 à 7, caractérisée en ce que le solvant organique est choisi parmi le toluène, le xylène, l'acétate d'éthyle, l'acétate de butyle, les cétones, les éthers de glycol, les esters, l'éthanol, l'isopropanol et le butanol, et leurs mélanges.

**9.** Utilisation selon l'une des revendications 1 à 5, caractérisée en ce que la base de soin ou le vernis à ongles est une composition en milieu aqueux.

**10.** Utilisation selon la revendication 9, caractérisée en ce que la composition comprend des particules de polymère filmogène en dispersion choisies parmi les polyuréthannes, les polyesters-polyuréthannes, les polyéther-polyuréthannes, les polymères radicalaires de type acrylique, acrylique styrène et acrylique vinylique, les polyesters, les résines alkydes, seuls ou en mélange.

**11.** Utilisation selon l'une quelconque des revendications précédentes, caractérisée en ce que la dispersion comprend également un polymère associatif de type polyuréthanne ou une gomme naturelle telle que la gomme xanthane.

**12.** Utilisation selon l'une quelconque des revendications précédentes, caractérisée en ce que la composition présente un taux de matière sèche de 25 - 45 % en poids.

**13.** Utilisation selon l'une quelconque des revendications précédentes, caractérisée en ce que la composition comprend également un agent plastifiant choisi parmi les citrates, les phtalates, les esters et/ou le camphre en une quantité de 5 - 30 % en poids par rapport au poids de la composition.

**Claims**

**1.** Use of colloidal silicic acid as curing agent in a varnish or nail care base composition comprising at least one film-forming material and at least one organic and/or aqueous and/or oily solvent.

**2.** Use of colloidal silicic acid as curing agent in a varnish or nail care base composition comprising at least one film-forming material and at least one organic and/or aqueous and/or oily solvent for increasing the lifetime of the varnish or base film.

**3.** Use of colloidal silicic acid as curing agent in a varnish or nail care base composition comprising at least one film-forming material and at least one organic and/or aqueous and/or oily solvent for improving the hold of a varnish or of a base and/or its resistance to rubbing, to wear and/or to impacts.

**4.** Use according to one of the preceding claims, in which the colloidal silicic acid is chosen from surface-treated silicas, hydrophilic pyrogenic silicas and/or hydrophobic pyrogenic silicas.

**5.** Use according to one of the preceding claims, in which the colloidal silicic acid is present in an amount of 0.05% to 5% by weight, preferably 0.5-2% by weight, with respect to the total weight of the composition.

**6.** Use according to one of the preceding claims, characterized in that the care base or varnish composition is a composition in organic solvent medium.

**7.** Use according to Claim 6, characterized in that the film-forming material is chosen from alkyd, acrylic and/or vinyl resins, polyurethanes and polyesters, celluloses and cellulose derivatives, such as nitrocellulose, and the resins resulting from the condensation of formaldehyde with an arylsulfonamide, and their mixtures.

**8.** Use according to one of Claims 6 and 7, characterized in that the organic solvent is chosen from toluene, xylene, ethyl acetate, butyl acetate, ketones, glycol ethers, esters, ethanol, isopropanol and butanol, and their mixtures.

9. Use according to one of Claims 1 to 5, characterized in that the care base or the nail varnish is a composition in aqueous medium.

10. Use according to Claim 9, characterized in that the composition comprises dispersed film-forming polymer particles chosen from polyurethanes, polyesters-polyurethanes, polyethers-polyurethanes, radical polymers of acrylic, acrylic-styrene and acrylic-vinyl type, polyesters or alkyd resins, alone or as a mixture.

11. Use according to any one of the preceding claims, characterized in that the dispersion also comprises an associative polymer of polyurethane type or a natural gum, such as xanthan gum.

12. Use according to any one of the preceding claims, characterized in that the composition exhibits a dry matter content of 25-45% by weight.

13. Use according to any one of the preceding claims, characterized in that the composition also comprises a plasticizing agent chosen from citrates, phthalates, esters and/or camphor in an amount of 5-30% by weight with respect to the weight of the composition.

**Patentansprüche**

1. Verwendung von kolloidaler Kieselsäure als Härtungsmittel in einer Lackzusammensetzung oder einer Pflegegrundmasse für die Nägel, die mindestens ein filmbildendes Material und mindestens ein organisches und/oder wäßriges und/oder öliges Lösungsmittel enthalten.

2. Verwendung von kolloidaler Kieselsäure als Härtungsmittel in einer Lackzusammensetzung oder einer Pflegegrundmasse für die Nägel, die mindestens ein filmbildendes Material und mindestens ein organisches und/oder wäßriges und/oder öliges Lösungsmittel enthalten, um die Lebensdauer des Films aus dem Lack oder der Grundmasse zu erhöhen.

3. Verwendung von kolloidaler Kieselsäure als Härtungsmittel in einer Lackzusammensetzung oder einer Pflegegrundmasse für die Nägel, die mindestens ein filmbildendes Material und mindestens ein organisches und/oder wäßriges und/oder öliges Lösungsmittel enthalten, um die Haltbarkeit eines Lacks oder einer Grundmasse und/oder ihre Beständigkeit gegenüber Reiben, Abnutzung und/oder Stöße zu verbessern.

4. Verwendung nach einem der vorhergehenden Ansprüche, wobei die kolloidale Kieselsäure unter den oberflächenbehandelten Kieselsäuren, den hydrophilen pyrogenen Kieselsäuren und/oder den hydrophoben pyrogenen Kieselsäuren ausgewählt ist.

5. Verwendung nach einem der vorhergehenden Ansprüche, wobei die kolloidale Kieselsäure in einem Mengenanteil im Bereich von 0,05 bis 5 Gew.-% und vorzugsweise im Bereich von 0,5 bis 2 Gew.-%, bezogen auf das Gesamtgewicht der Zusammensetzung, vorliegt.

6. Verwendung nach einem der vorhergehenden Ansprüche, dadurch gekennzeichnet, daß die Zusammensetzung für die Pflegegrundmasse oder den Lack eine Zusammensetzung in einem organischen Lösungsmittelmedium ist.

7. Verwendung nach Anspruch 6, dadurch gekennzeichnet, daß das filmbildende Material unter den Alkydharzen, Acrylharzen und/oder Vinylharzen, Polyurethanen und Poiyestern, Cellulosen und Cellulosederivaten, wie Nitrocellulose, den Harzen, die bei der Kondensation von Formaldehyd mit Arylsulfonamiden entstehen, und deren Gemischen ausgewählt ist.

8. Verwendung nach einem der Ansprüche 6 bis 7, dadurch gekennzeichnet, daß das organische Lösungsmittel unter Toluol, Xylol, Ethylacetat, Butylacetat, Ketonen, Glykolethern, Estern, Ethanol, Isopropanol und Butanol sowie deren Gemischen ausgewählt ist.

9. Verwendung nach einem der Ansprüche 1 bis 5, dadurch gekennzeichnet, daß die Pflegegrundmasse oder der Nagellack eine Zusammensetzung in wäßrigem Medium ist.

10. Verwendung nach Anspruch 9, dadurch gekennzeichnet, daß die Zusammensetzung Partikel eines filmbildenden Polymers in Dispersion enthält, das unter Polyurethanen, Polyester-Polyurethanen, Polyether-Polyurethanen, durch radikalische Polymerisation hergestellten Polymeren vom Acryltyp, Acryl-Styroltyp und Acryl-Vinyltyp, Polyestern, Alkydharzen sowie deren Gemischen ausgewählt ist.

11. Verwendung nach einem der vorhergehenden Ansprüche, dadurch gekennzeichnet, daß die Dispersion ferner ein assoziatives Polymer vom Polyurethantyp oder ein natürliches Gummi, wie Xanthangummi, enthält.

12. Verwendung nach einem der vorhergehenden Ansprüche, dadurch gekennzeichnet, daß die Zusammensetzung einen Trockensubstanzgehalt von 25 bis 45 Gew.-% aufweist.

13. Verwendung nach einem der vorhergehenden Ansprüche, dadurch gekennzeichnet, daß die Zusammensetzung ferner einen Weichmacher enthält, der unter den Citraten, Phthalaten, Estern und/oder Campher in einem Mengenanteil im Bereich von 5 bis 30 Gew.-%, bezogen auf das Gesamtgewicht der Zusammensetzung, ausgewählt ist.